Europäisches Patentamt

European Patent Office

Office européen des brevets

(11) Publication number: **0 118 274**
**A1**

# (12) EUROPEAN PATENT APPLICATION

(21) Application number: **84301273.3**

(22) Date of filing: **27.02.84**

(51) Int. Cl.³: **C 12 Q 1/04**
**C 12 Q 1/18, C 12 Q 1/34**

(30) Priority: **25.02.83 GB 8305324**

(43) Date of publication of application:
**12.09.84 Bulletin 84/37**

(84) Designated Contracting States:
**AT BE CH DE FR GB IT LI LU NL SE**

(71) Applicant: **UNILEVER PLC**
**Unilever House Blackfriars P O Box 68**
**London EC4P 4BQ(GB)**

(84) Designated Contracting States:
**GB**

(71) Applicant: **UNILEVER NV**
**Burgemeester s'Jacobplein 1 P.O. Box 760**
**NL-3000 DK Rotterdam(NL)**

(84) Designated Contracting States:
**BE CH DE FR IT LI LU NL SE AT**

(72) Inventor: **Carr, Anthony Hugh**
**28 Manor Farm Way**
**Sharnbrook Bedford(GB)**

(72) Inventor: **Jobling, Ian**
**25 Lynford Way**
**Rushden Northamptonshire(GB)**

(74) Representative: **Stancliffe, Terence Christopher et al,**
**UNILEVER PLC Patents Division P.O. Box 68 Unilever**
**House**
**London EC4P 4BQ(GB)**

(54) Microbiological test processes and apparatus.

(57) Microbiological test processes and apparatus for iden-
tifying producers of penicillinase, in which a container for
test incubation of a microorganism culture suspension with a
cell density about $10^8$ per ml is provided with penicillin, poor
buffering and a fluorescent indicator, e.g. a coumarin such as
4-methyl-umbelliferone, in quantity sufficient to discriminate
penicillinase producers and non-producers after incubation
times over about one hour, e.g. up to 18 hours. The tests can
then be conveniently combined with antibiotic sensitivity
testing, e.g. in different microtitre wells of the same micro-
titre plate.

Croydon Printing Company Ltd.

## Microbiological Test Processes and Apparatus

This invention relates to microbiological test processes and apparatus.

It is now well-known in clinical laboratory practice to carry out microbial tests to establish the susceptibility or resistance of microorganisms isolated from septic clinical samples, such as urine, wound exudates or blood, to each of a variety of potentially therapeutic antimicrobial agents. The purpose is to provide a basis on which clinical therapeutic decisions can be taken.

The original antibiotic susceptibility test of the MIC type, to determine minimum inhibitory concentrations of the antimicrobial agents under test in relation to the specimen microorganisms from the patient under investigation, generally require initial culture of the septic sample to provide an inoculum for the MIC tests themselves, followed by further culture in the MIC test media. Such further culture is commonly carried on for 14-18 hours, e.g. overnight.

A number of attempts have been made to alter the conditions under which the sensitivity tests are carried out, in order to provide a culture result, and an indication of the sensitivity of the microorganisms to the antimicrobial substances under test, in a shorter period of time.

Examples of microbial tests embodying such attempts are published in USP 3 509 026 (Litton Systems Inc.), USP 4 236 211 (Pfizer), USP 3 832 532 (Pfizer) and GB 1 554 134 (Pfizer).

In our experience one notable drawback is associated with attempts to carry out MIC testing using shorter periods of culture incubation, e.g. less than 8 hours, and sometimes as little as 4-5 hours. This drawback occurs in many cases of microorganisms which show resistance to penicillin. Such organisms form a beta-lactamase enzyme that converts penicillin to an inactive form. However, in many cases the full effects of the beta-lactamase enzyme in combating the effect of penicillin only show themselves several hours after the start of the culture period. The result of this is that microbial culture tests that rely on desirably short periods of microbial culture incubation can give a misleadingly optimistic account of the longer-term sensitivity of the microorganisms to penicillin. This problem is especially acute with clinical isolates of Staphylococcus aureus.

It is known to test microorganisms for their ability to produce beta-lactamase enzyme, and in particular by exposing a culture of the microorganism to penicillin itself and detecting the penicilloic acid produced. However, such methods have not been rendered suitable for carrying out directly in conjunction with antibiotic sensitivity-testing methods. For example, one prior test, known commercially as the "Intralactam" test (Trade Mark), involves rubbing or spreading an undilated microorganism culture (e.g. with a loop touched on a number of microbial colonies) on to a moistened paper strip impregnated with penicilloic acid and a purple-yellow colour-change material sensitive to acid production, and incubating the paper for some minutes.

According to the present invention we provide a convenient arrangement of a microbiological test process and apparatus for identifying microorganisms which are producers of beta-lactamase enzyme, in conjunction with antibiotic sensitivity testing, and we thereby enable more reliable use to be conveniently made of antimicrobial sensitivity tests employing short culture periods; e.g. of about 5 hours, as well as longer tests using culture periods, e.g. of about 18 hours.

In tests arranged according to the present invention, a diluted suspension of the microorganism is incubated in a suitable container such as a microtitre well in a multiwell microtitre plate of standard design, with an excess of penicillin, in a medium which is poorly buffered against changes of pH, in the presence of a substance capable of indicating the development of acidity by alteration of fluorescence, for an incubation period of more than about one hour, for example about 1-4 hours, and possibly for longer, e.g. about 5 hours, or alternatively for example up to about 18 hours.

The excess of penicillin used is one that is capable of significantly lowering the pH of the medium when it is hydrolysed during the incubation period by action of the beta-lactamase present in the inoculum, forming penicilloic acid and causing a detectable change in the state of the indicator. Suitably, quantities of the order of 0.1% to 0.5% penicillin by weight can be used. At the same time, it is important not to use excess penicillin, otherwise negative cultures can give false positive results due to spontaneous hydrolysis of penicillin or any other reason.

The medium used is selected to have a poor buffering capacity for pH, and can be for example water, a dilute aqueous polyvinylalcohol solution, or peptone medium, or a dilute phosphate solution.  The degree of buffering needed or tolerated will be greater as the incubation time is greater.  For incubations of 5 hours to 18 hours a buffer capacity equivalent to about .1-5 mM phosphate is suitable, with initial pH for example about 9.2.  This arrangement can allow discrimination of lactamase producers and non-producers after incubation times in the range 5 hours to 18 hours.

Convenient inoculum levels are for example of the order of $10^8$ organisms per ml.  One advantage of the invention in comparison with earlier tests is that the microorganism suspension which is used for the test can be a sample from a suspension which is further diluted in nutrient broth to form an inoculum for antibiotic sensitivity testing, thus ensuring identity of the subjects of the two tests.  A further advantage of tests arranged according to the invention is that the incubations can last similar times and the results of the two forms of tests can be read off on a single occasion and by similar fluoroscopic or fluorometric means.

The indicator used can be for example an indicator showing a fluorescence which is either enhanced from a low level, or quenched from a high level, upon acidification.

One very suitable example of an indicator is a fluorescent coumarin derivative, e.g. 4-methyl-umbelliferone: its fluorescence becomes quenched with a fall in pH due to acidification.

It is important that the $pK_a$ of the indicator is not excessively higher than the starting pH of the incubation. This starting pH can conveniently be in the range 7-8, or up to 9.5., e.g. about 9.2, and the $pK_a$ of the indicator is preferably in the range 6.5-8.5.  The $pK_a$ of the 4-methyl-umbelliferone is at 7.9.

Preferably, the result of the test is read fluorimetrically.  This provides a graduated scale result and can distinguish between strong and weak beta-lactamase producers.

When the test is carried out for example in the way described below, specimens showing a significant lowering of the 4-methyl-umbelliferone fluorescence upon incubation can be taken as probable beta-lactamase producers.

According to the invention, apparatus for the performance of the tests described herein comprises a container for incubation of a microorganism, containing excess penicillin and a substance capable of detectably indicating the development of acidity by alteration of its fluorescence, in the absence of materials capable of exerting an appreciable buffering capacity of the pH of the test incubation.

These materials can very conveniently be present in dried stabilised form, for example incorporated in a dried film adhering to an inner surface of the container, for example comprising a (non-buffering) film-forming stabiliser, such as polyvinyl alcohol.  A suitable stabilisation method is described for example in GB 1 574 707 (Unilever).

Conveniently the container for the tests can be a well of a microtitre tray.  Commonly-used such trays contain a number of microtitre wells, commonly of about 0.3-0.5 ml capacity, and these are conveniently usable for the present test with about 50 microlitres of incubation material in each well.

In certain convenient embodiments, apparatus according to the invention comprises the test containers as individual (e.g. duplicate) microtitre wells containing stabiliser, penicillin and indicator, in a microtitre tray of which the remaining wells are fitted up for MIC testing, for example in a manner known per se. Alternatively, the indicator can be absent from the dry plate and added to and with the inoculum.

It can also be seen that in these embodiments, the invention provides a process of testing a microorganism for the presence and/or extent of the property of producing beta-lactamase, in conjunction with a test of the antibiotic sensitivity of the same microorganism; which comprises (a) making a dilution of a culture of the microorganism to form a suspension, part of which is tested for beta-lactamase production in the manner described herein, and (b) diluting another part of the suspension in nutrient broth to form an inoculum which is then used to carry out antibiotic sensitivity testing of the microorganism.

Preferably the same indicator method is used to read the results of both forms of testing. For example, the beta-lactamase production can be estimated by fluorimetry of a suitable fluorophor, for example 4-methyl-umbelliferone as above, and the growth of the microorganism can be estimated by fluorimetry of a suitable similar fluorophor such as 4-methyl-umbelliferone and/or 7-amino-4-methyl coumarin released by hydrolysis of corresponding hydrolysable fluorogenic enzyme substrates such as phosphate, nonanoate or alanine esters or peptide upon growth of the microorganism.

The invention is illustrated by the following examples.

Example 1

100 ml of sterile 0.5% polyvinylalcohol solution, containing 204.8 mg penicillin G sodium salt and 3.52 mg 4-methyl-umbelliferone was made up and applied in 25 microlitre aliquots to 0.5 ml plastics microtitre wells not otherwise occupied in a microtitre tray being prepared for MIC testing, using the procedure of GB 1 574 707 to give a stabilised dry product.

Suspensions of three unidentified clinical isolate microorganisms were obtained after their independent characterisation, by conventional methods for comparative purposes, as a beta-lactamase non-producer, weak producer and strong producer respectively. The non-producer appeared similar to a known common laboratory reference strain of non-betalactamase-producing Staphylococcus aureus 'Oxford'.

Each microorganism was formed into a suspension inoculum in peptone medium to a cell density of about $10^8$ per ml. 50 microlitres of one of each of the three inocula was inoculated into a respective microtitre well prepared as described above, and incubated for 4 hours at 37°C.

The initial flurometric values of the inoculated mixtures in the test wells ($F_o$) and the fluorimetric values after 4 hours incubation ($F_4$) were as follows:

|  |  | $F_o$ | $F_4$ |
|---|---|---|---|
| Betalactamase non-producer | | 2500 | 2500 |
| " | weak producer | 2500 | 1800 |
| " | strong producer | 2500 | 850 |

The test results were seen to give good discrimination between producers and non-producers of beta-lactamase.

The tests were carried out in conjunction with antibiotic sensitivity testing of the same microorganisms. For this purpose, another portion of each suspension was further diluted to a concentration in the range $5 \times 10^4$ to $10^6$ organisms per ml in nutrient broth arranged to contain fluorogens hyrdolysable upon growth of the microorganisms, in this case 100-120 micromolar concentration of each of 4-methyl-umbelliferone phosphate, the corresponding nonanoate, and the L-alanyl-peptide of 7-amino-4-methyl-coumarin, all three previously dried down as a dry stable film with a stabiliser such as polyvinylalcohol on a surface of the container used to prepare the inoculum broth suspension or a closure, e.g. a plastics lid, or a solid non-porous-surface e.g., insert therefor. The broth suspension was dosed into those wells of the MIC plate which had been prepared with dry stable films containing appropriately graded doses of antibiotic according to the standard technique.

The whole microtitre plate, including MIC wells and beta-lactamase test wells, was then incubated for 4 hours, and the results (growth/no growth, or lactamase/weak or no lactamase respectively) were estimated by fluorimetry using a fluorimeter working at 363 nm (excitation) and 450 nm (detection) and arranged with photomultiplier detector calibrated and backed-off in known manner to read fluorescence values corresponding to concentrations of 4-methyl-umbelliferone fluorophor in the range 0-3 and up to 14 micromolar concentration.

Example 2

A penicillin-containing fluorescent lactamase indicator composition to be applied in 25 microlitre aliquots as in Example 1 contained 5 g/l polyvinylalcohol, 140 mg/l 4-methyl-umbelliferone, 2 g/l penicillin G, 0.1% commercially available microbiological peptone, and 4 mM disodium phosphate.

When the indicator was applied and used as described in reference to Example 1 it was found that satisfactory results in regard to discrimination of lactamase-positive and lactamase-negative microbial inocula could be obtained either after 5 hours' or after 18 hours' incubation of the wells.

Example 3

A further penicillin-containing fluorescent lactamase indicator composition contained 0.27M sodium chloride, 1.5 g/l penicillin G, 0.45 g/l disodium phosphate and 40 mg/l 4-methyl-umbelliferone, optionally with polyvinyl alcohol as described above. The starting pH is adjusted to 9.2.

P140 **0118274**

When applied and used as described in reference to Example 1 similar results were obtained as with Examples 1 and 2.

It can be seen that the invention is susceptible of many modifications apparent to the skilled reader in the light of the foregoing description, and any and all combinations of the several features described above are intended within the scope of the following claims.

Claims

1.  A microbiological test process for identifying microorganisms which are producers of beta-lactamase enzyme (penicillinase), which comprises incubating the microorganism under test with penicillin and an acid-base indicator, characterised in that the test is carried out by incubating an aqueous suspension of microorganisms, preferably containing of the order of $10^8$ organisms per millilitre, in a container with a test medium poorly buffered against changes of pH and in the presence of an indicator substance capable of indicating the development of acidity by alteration of fluorescence, for an incubation time generally in excess of about one hour, e.g. 1-4 hours, or about 5 hours, or about 18 hours.

2.  A process according to claim 1, characterised in that the test is carried out in the presence of a fluorophor showing reduced fluorescence in the presence of acid produced by the microorganism by hydrolysis of penicillin.

3.  A process according to claim 2, characterised in that the fluorophor is a fluorescent coumarin derivative, for example 4-methyl-umbelliferone.

4.  A process according to claims 1, 2 or 3, characterised in that the poor buffering of the medium is sufficient to allow discrimination of lactamase positive and negative microorganisms after incubation times in the range about 5 hours to 18 hours.

5.  A process according to claim 4, characterised in that the test medium contains buffering equivalent to a phosphate concentration in the range about 1-5 millimolar and an initial pH about 9.2.

6.    A process according to any of claims 1-5, characterised in that the microorganism of which a suspension is tested is also subjected to a culture test to determine its antibiotic sensitivity, over a similar period of time as the period used for the test for beta-lactamase.

7.    A process according to any of claim 1-5, characterised in that the test is carried out in a well of a microtitre plate of which other wells are used for carrying out antibiotic sensitivity testing of the same microorganism, and the microorganisms for both tests are sampled from a common suspension to ensure their identity with each other.

8.    A microbiological test apparatus for identifying microorganisms which are producers of beta-lactamase enzyme (penicillinase), which comprises a carrier with penicillin and an acid-base indicator, characterised in that the test apparatus comprises a container for containing a suspension of the microorganism under test, and preserved in dry form in the container reagents for a test medium which is poorly buffered against changes in pH, including penicillin and an indicator capable of indicating the development of acidity by alteration of fluorescence, in quantity sufficient to allow discrimination of lactamase producers and non-producers after incubation times in excess of about one hour, e.g. 1-4 hours, or about 5 hours, or up to about 18 hours.

9.    A microbiological test apparatus according to claim 8, further characterised by any one or more of the features specified in claims 2 to 7.

10.   Microbiological test processes and apparatus according to claims 1 and 8, characterised by any one or more of the several features of the foregoing description and examples.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,Y | US-A-4 390 622 (G.W. CARTWRIGHT) * Whole document * | 1 | C 12 Q 1/04<br>C 12 Q 1/18<br>C 12 Q 1/34 |
| A | | 6 | |
| Y | CLINICAL CHEMISTRY, vol. 27, no. 9, September 1981, pages 1490-1498, Easton, Pennsylvania, US<br>R.H. YOLKEN: "Enzymic analysis for rapid detection of microbial infection in human body fluids: an overview" * Pages 1490-1492 * | 1 | |
| A | EP-A-0 037 932 (TEVA PHARMACEUTICAL INDUSTRIES LTD.) * Whole document * | 1,6,8 | TECHNICAL FIELDS SEARCHED (Int. Cl. ³) |
| A | EP-A-0 059 645 (NATIONAL RESEARCH DEVELOPMENT CORP.) | | C 12 Q |
| A | EP-A-0 005 891 (GIST-BROCADES N.V.) | | |

The present search report has been drawn up for all claims

| Place of search | Date of completion of the search | Examiner |
|---|---|---|
| THE HAGUE | 18-05-1984 | GRIFFITH G. |